Europäisches Patentamt

(19) European Patent Office

Office européen des brevets

(11) Publication number: **0 171 496 B1**

# EUROPEAN PATENT SPECIFICATION

(45) Date of publication of patent specification: 26.05.93

(51) Int. Cl.5: **C12N 15/00**, C07K 15/00, C12P 21/00, C12N 5/00

(21) Application number: 85102665.8

(22) Date of filing: 08.03.85

(54) Process for the production of a chimera monoclonal antibody.

(30) Priority: 15.08.84 JP 169370/84

(43) Date of publication of application:
19.02.86 Bulletin 86/08

(45) Publication of the grant of the patent:
26.05.93 Bulletin 93/21

(84) Designated Contracting States:
CH DE FR GB LI SE

(56) References cited:
EP-A- 0 125 023

PROCEEDINGS OF THE NATIONAL ACADEMY
OF SCIENCES OF THE USA, vol. 81, June
1984, pages 3273-3277; S. CABILLY et al.:
"Generation of antibody activity from im-
munoglobulin polypeptide chains produced
in Escherichia coli"

NATURE, vol. 309, 24th May 1984, pages
364-369; J. SHARON et al.: "Expression of a
VhCk chimaeric protein in mouse myeloma
cells"

Genes, Benjamin Lewin, Editor Cell, 1983,
Jon Wiley & Sons, N.Y. Ch. 39

(73) Proprietor: **Research Development Corpora-
tion of Japan**
5-2, Nagatacho 2-chome
Chiyoda-ku Tokyo(JP)

(72) Inventor: **Taniguchi, Masaru**
17-12, Konakadai 3-chome
Chiba-shi Chiba-ken 281(JP)
Inventor: **Kurosawa, Yoshikazu Lions Mansion
2-215**
20-1, Fujimigaoka Yagoto Tempaku-cho
Showa-ku Nagoya-shi Aichi-ken 468(JP)
Inventor: **Sugita, Kou-zou**
Shoh-nan-so 1-60, Hiokamachi Chikusa-
ku
Nagoya-shi Aichi-ken 464(JP)

(74) Representative: **Kraus, Walter, Dr. et al**
Patentanwälte Kraus, Weisert & Partner
Thomas-Wimmer-Ring 15
W-8000 München 22 (DE)

**Description**

This invention relates to a production process of a chimera monoclonal antibody. More particularly, this invention concerns the production of a chimera monoclonal antibody whose side – effects such as ana – phylaxis and serum sickness are thought to be greatly reduced especially when it is administered to man.

A monoclonal antibody with mono – specificity has given a great influence to immunology and its usefulness has already been demonstrated over such sciences as biology, pharmacology, chemistry and others.

Concerning its production process, Kohler and Milstein succeeded in realizing in 1975 by fusing mouse spleen cell primed with sheep erythrocytes and mouse melanoma cell (See Nature; 256, 495 – 497 (1975) – .). Outside this, there is another one utilizing Epstein – Barr virus (See Japanese Patent publication Laid Open No. sho 58 – 201723.).

However, since most of monoclonal antibodies are derived from animals except man, they are expected to cause such side – effects as anaphylaxis or serum sickness when administered to man because heterologous proteins are injected into a human body. Accordingly, various attempts have so far been made to manufacture human – derived monoclonal antibodies by using human hybridoma. Among them are Japanese Patent Publication No. sho 57 – 126424, sho 57 – 502090, sho 58 – 90517, sho 58 – 128323 and sho 57 – 50209 for example.

These attempts tell us that the production of human – derived monoclonal antibodies is certainly possible but it is still insufficient in view of its reproducibility and others. (See Nature; 300, 316 – 317 (1982).). In addition, there is such disadvantage that immunization of man with any desired antigen is impossible although immunization of animals like mice with various antigens is possible.

In this respect, there is an attempt to cause coliform bacillus to produce a human – derived monoclonal antibody by inserting a complementary DNA into plasmid pBR 322. The cDNA was made by p – chain specific m RNA obtained from human mouse hybridoma that produces human monoclonal antibodies. However, since it is unable to immunize man with various antigens so well as desired, it must be said that there still remains a problem.

EP – A – 0 125 023 discloses a method for producing chimeric antibodies and immunoglobulins, respectively, which are analogous to those normally found in vertebrate systems.

Proc. Natl. Acad. Sci. USA, Vol. 81, pp. 3273 – 3277, June 1984, discloses the construction of plasmids from H and/or L chains of CEA antibodies directly produced in E. coli.

Under the circumstances, the present inventors made various studies to eliminate the above drawbacks and finally have accomplished this invention. That is, this invention concerns a production process of chimera monoclonal antibody consisting of a variable region derived from mouse and a constant region derived from man, which is characterized by comprising (a) inserting into an expression vector active genes: $V_H$ and $V_L$, isolated from hybridomas as antibody – producing cells of mouse and genes: $C_H$ and $C_L$, isolated from human DNA, and then (b) introducing an expression vector from (a) into lymphoma as cultured animal cells.

It is an object of this invention to provide a production process of a chimera monoclonal antibody whose side – effects such as anaphylaxie shock and serum sickness are greatly reduced by combining a variable region derived from mouse and a constant region derived from man because most of conventional monoclonal antibodies are derived exclusively from animals except man and expected to be a cause of such side – effects as anaphylaxis shock and serum sickness upon administering to a human body on account of being heterologous proteins.

This production process can be industrialized offhand with a reduced cost and safety.

The above and other objects and features of this invention will appear more fully hereinafter from a consideration of the following description taken in connection with the accompanying drawing wherein one example is illustrated by way of example.

Brief Description of the Drawings

Fig. 1 is x – ray photographs showing the result of the southern hybridization carried out to isolate active mouse V gene and human C gene, and m RNA northern blotting analysis thereof; Fig. 1B illustrates schematically the various clones isolated from hybrydoma and human gene library, where (a) illustrates clone $VJ_x$ 14, (b) clone $VJ_H$ 243, (c) clone $HC_x$ 2, and (d) clone HG 163

Fig. 2 shows plasmid structures formed to use for DNA transformation, where (a) illustrates plasmid $pSV2 – HC_kV_{D10}$ structure and (b) plasmid $pSV2 – HGIV_{D10}$ structure;

Fig. 3 is diagrams of FACS analysis and

Fig. 4A is x−ray photographs showing the result of DNA southern hybridization of HMH cells; Fig. 4B is x−ray photographs showing the result of m RNA northern blotting analysis.

The term "active $V_H$ and $V_L$ genes" herein, is meant to express functional genes which have the structure V−D−J with regard to $V_H$ and the structure V−J with regard to $V_L$, provided $V_H$ and $V_L$ are formed by the DNA rearrangement in antibody−producing cells.

Hybridomas, cloned B cells and B cells transformed with Epstein−Barr virus are employed preferably as the "antibody−producing cells; and vectors pSV2−gpt, pSV2−neo and SV40 are favorably used as the "expression vector".

Moreover, any one of lymphoma cells, kidney cells, L cells, COS cells and HeLa cells derived from mouse can be used as the "cultured animals cells".

In the meantime, according to the present invention, mice are so easy to immunize that any desired chimera monoclonal antibody can be obtained; at the same time, it is naturally expected that the antigenicity of heterologous immunogloblin proteins is greatly reduced in the use of the chimera monoclonal antibody of this invention, compared with other derived from animals only.

The present invention will be described in more detail with reference to the following example.

Example

Isolation of Mouse V Gene

Hybridoma D10, formally designated M2590, is the fused cell between the recipient tumour cell, P3U1, and the spleen cell derived from the C57BL/6 mouse which were immumized with B−16 melanoma cell that had been generated spontaneously in C57BL/6 mouse. D10 secretes the antibody which veacts with melanoma cells selectively. The type of this antibody is IgM for heavy chain and Kappa for light chain.

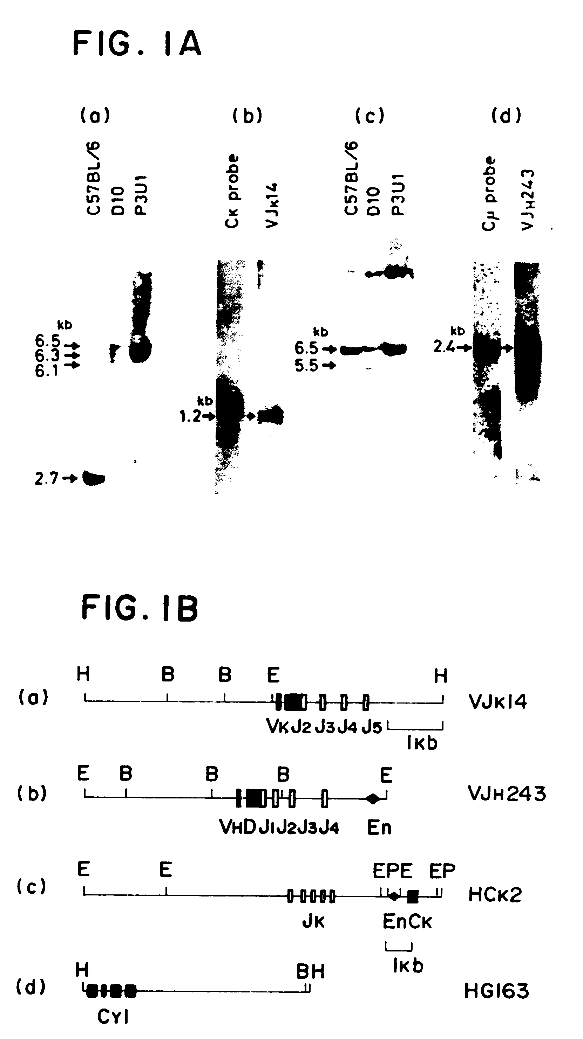

At first, DNA is isolated from D10, P3U1 and C57BL/6 mouse kidney, respectively (See Cell; 24, 353 − 356 (1981).). Next, 10 $\mu$g of DNA is digested with the restriction emzyme. Hind III and EcoRI. D10, P3U1, and C57BL/6 mouse−derived DNA digested with Hind III are developed into 0.9% agarose gel and transferred to the nitrocellulose membrane (See Schleicher and Schvell; J. Mol. Biol., 98, 503 − 515 (1975) −.). On the other hand, hybridization is carried out with $J_x$ probe ($10^7$ cpm/0.1 $\mu$g DNA) which corresponds to 2.7 Kb Hind III−Hind III fragment containing $J_x$ region which was given by Mr. Susumu Tonegawa (See Nature; 280, 288 − 294 (1979).). The result is shown in Fig. 1A(a).

As is apparent from Fig. 1A(a), there exist three rearranged bands at 6.5, 6.3 and 6.1 Kb in D10 DNA. The two bands at 6.3 and 6.1 Kb are the same as the one found in P3U1 DNA. The band at 6.5 Kb is an active gene containing the $V_x − J_x$ structure. The gene is responsible for its antigen specificity. The size of DNA is estimated by the use of the λ piage Hind III marker. The DNA gragments which corresponds to this size are isolated by the agarose gel electrophoresis, inserted into λ phage Hind III vector λ 788, which was given by Mr. K. Murray of Edingburph University (See Mole. Gen. Genet; 150, 53 − 61 (1977).) and then packaged into λ phages. Coliform bacilli BHB 2688 and BHB 2690 are used as a packaging mixture (See Hohn. B. Meth. Enzymol.; 68, 299 − 309 (1979).). Next, Plaque hybridization is carried out according to the Benton−Davis method (Science, 196, 180 − 182 (1977) by using $J_x$ probe for screening. The clone $VJ_x14$ is isolated. The restriction map of the clone is given in Table 1B(a).

The Hind III−inserted $VJ_x$ fragment is isolated to carry out northern hybridization. Total RNA is isolated from D10 according to the guanidinium thiocyanate method (Biochemistry; 18, 5294 − 5299 (1979)); after that, Poly A containing m RNA is obtained from fractions that have passed through the oligo dT cellulose column. Fig. 1A(b) shows the northern hybridization of m RNA plus the Hind III−inserted clone $VJ_x$ 14 fragment or m RNA plus $C_x$ probe which correspnds to 3Kb Hind III−BamHI fragment containing $C_x$ region, which was given by Mr. Susumu Tonegawa (See Nature; 280, 288 − 294 (1979).). A band is observed at 1.2 Kb by the two probes $J_x$ and $C_x$. The clone $VJ_x$ 14 contains the functional $V_x − J_x$ structute. How to carry out the northern hybridization is described in Immunological Experimental Procedures XII (1983).

Fig. 1A(c) shows the southern hybridization of $J_H$ probe which corresponds to XbaI − EcoRI fragment at 0.9 Kb (Cell.; 24, 353 − 365 (1981)) and DNA digested with EcoRI.

Based on the reason mentioned above, DNA at 5.5 Kb, which is found only in D10 which contains a functional gene in variable region of H chain is cloned by λ phage EcoRI vector λgt WES−λB, (See P. Leader, Science; 196, 175 − 177 (1977).) and the clone $VJ_H$ 243 is obtained. Clone $VJ_H$ 243 and $C\mu$ probe which corresponds to 10 Kb EcoRI fragment of MEP 203 (Proc. Natl. Acad. Sci. USA: 77, 2138 − 2142 (1980)) are used to make northern blotting analysis with m RNA of D10. As a result, a band is found at 2.4 Kb (See Fig. 1A(d).). V gene contained in the clones $VJ_\lambda$ 14 and $VJ_H$ 243 is related to their autigen specificity.

Isolation of Human C Gene

The C gene of the IgG$_1$ class which is the major immunogloblin class in human blood serum is isolated. Inasmuch as the necleotide sequences in genes of human immunogloblin has a great resemblance to that of mouse immunogloblin, C$_x$ and C$_{\gamma 1}$ genes present in human genomes are intended to isolate by using correspondent mouse genes as a probe. Namely, Hind III − BamHI fragments (3 Kb) from the clone Ig 146 (Proc. Natl. Acad. Sci. USA; 78, 474 − 478 (1981)) are used as probe to isolate fragments which contains C$_x$ gene together with an enhancer region from the Hae III − Alu I gene library in λ charon 4A (T. Maniatis, Cell.; 15, 1157 − 1174 (1978)). The C$_{\gamma 1}$ genes are obtained by digesting DNA from human fetus liver cells with Hind III, by fractionating resulting DNA fragments through agarose gel electrophoresis in order of their size, by inserting a fragment at 5.9 Kb into λ 788 and finally by cloning it with the avoe probe. The clones isolated are HC$_x$2 shown in Fig. 1B(c) and HG 163 shown in Fig. 1B(d).

Preparation of $_p$SV2 − HC$_x$V$_{10}$ Plasmid Containing Mouse V$_x$ Gene and Human C$_x$ Gene

P Pvu II fragment at 1.9 Kb which contains an enhancer elements and human C$_x$ gene is isolated from the clone HC$_x$2 shown in Fig. 1B(c).

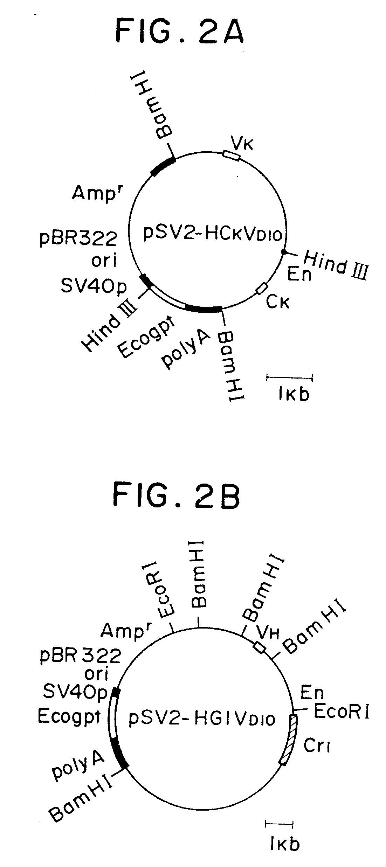

After a 50/50 mixture of Hind III and BamHI linker (produced by Takara Shuzo Co., Ltd.) has been ligated to it, the built − up product is digested with Hind III. Fragment thus obtained is ligated with a Hind III − inserted fragment at 6.5 Kb which is isolated from VJ$_x$ 14 and then the resulting product is digested with BamHI into fragments which are subjected to fractionation. A fragment at 5.9 Kb is isolated by agarose gel electrophoresis. The isolated fragment is inserted into $_p$SV2gpt at BamHI site. The direction of the inserted gene is determined by the restriction map (See $_p$SV2 − HC$_k$V$_{D10}$ in Fig. 2(a).).

Preparation of pSV2 − HG1V$_{D10}$ plasmid Containing Mouse V$_H$ Gene and Human C$_{\gamma 1}$ Gene

Fragment with 8.2 Kb Kind III insert is isolated from HG 163 clone. Both its ends are converted to blunt end with Klenov enzyme and digested with EcoRI linker (produced by Takara Shuzo Co., Ltd.). After that, the fragment is digested with EcoRI and BamHI and inserted into EcoRI − and BamHI − cleaved plasmid pSV2 − gpt. In this way, the clone, pSV2 − HG14, containing human C$_{\gamma 1}$ gene is obtained. 5.5 Kb EcoRI fragment is isolated from the clone VJ$_H$243 and inserted into the EcoRI site of pSV2 − HG14. The orientation of the inserted gene is determined by the restriction map. (See PSV2 − HGIV$_{D10}$ in Fig. 2(b).)

Transformation of Plasmacytoma by Means of the Plasmids pSV2 − CH$_k$V$_{D10}$ and pSV2 − HC$_1$V$_{D10}$

Both DNAs: pSV2 − HC$_k$V$_{D10}$ and pSV2 − HG1V$_{D10}$ are introduced into the mouse plasmacytoma P3U1 by the calcium phosphate co − precipitation method (Proc. Natl. Acad. Sci. USA: 76, 1373 − 1376 (1979)). The method comprises the following steps.

(1) Solution A is added to the equal amount of the solution 2xHeBS drop by drop.

(2) The mixture is incubated at room temperatures for 30 minutes.

(3) The plasmacytoma P3U1 is treated with trypsin as far as it dissociate into pieces. After that, the culture medium RPMI 1640 containing 10 % of fetal calf serum is added thereto in order to terminate the trypsin treatment

(4) The culture medium is subjected to centrifugation at 1,500 rpm for 5 minutes to collect the cells.

(5) The cells ($2 \times 10^7$) are added to the culture medium without fetal calf serum drop by drop.

(6) The mixture is centrifuged at 1,500 rpm for 5 minutes.

(7) The cells are suspended in the solution prepared in the 1st step.

(8) The resulting mixture is incubated at 37 ˚C for 30 minutes.

(9) 5 ml of it transfer to another test tube.

(10) Culture medium RPMI 1640 containing 10 % of fetal calf serum (45 ml) is added to the test tube.

(11) The cell suspension (0.1 ml) is put in 96 wells on a culture plate in which the cells may be divided by $2 \times 10^4$ in number.

(12) The cells are cultured for 72 hours with the culture medium RPMI 1640 containing 10 % of fetal calf serum.

(13) The culture medium is replaced with the culture medium RPMI 1640 which contains 5 $\mu$g/ml of mycophenolic acid and 250 $\mu$g/ml of xanthine, together with 10 % of fetal calf serum in order to select transformed cells.

The solution A and the solution 2xHeBS have the following composition.

<u>Solution A</u>

| | | |
|---|---|---|
| $pSV2-HG1V_{D10}$ | 1140 µl | (containing 200 µl of plasmid) |
| $pSV2-HC_kV_{D10}$ | 900 µl | ( do. ) |
| 2 M $CaCl_2$ | 312.5 µl | (Sterilized in an autoclave) |
| Re-distilled $H_2O$ | 2647 µl | |

<u>Solution 2xHeBS</u>   pH 7.05

| | | |
|---|---|---|
| HEPES | 10 | g/l |
| NaCl | 16 | g/l |
| KCl | 0.74 | g/l |
| $Na_2HPO_4H_2O$ | 0.25 | g/l |
| dextrose | 2 | g/l |

Select of a Transformed Cell That Produces a Chimera Monoclonal Antibody

In order to select a transformed cell producing a chimera monoclonal antibody, the enzyme − linked antibody method and the analysis by means of the cell Sorter, FACS (a product of Beckton − Deckinson Co., Ltd.), are employed. Eighteen clones of transformed cells are selected by the use of a selective culture medium which contains mycophenolic acid. The plasmacytoma P3U1 and the eighteen clones of trans − formed cells are kept on growing to the full in a plate containing the selective culture medium. Supernatant of these culture media are subjected to the enzyme − linked antibody method (Meth. Enzymol.; 70, 419 − 439 (1980)) to check their antibody − producing ability. The result is given in Table 1

Table 1

| Cell | Antibody | |
|---|---|---|
| | Rabbit anti − human IgGFc antibody | Rabbit anti − human $C_x$ antibody |
| HMH − S1 | − | − |
| HMH − S6 | − | − |
| HMH − S7 | + | + |
| HMH − S8 | − | − |
| HMH − S18 | − | − |

It becomes clear that $1 \times 10^7$ HMH − S7 cells secreate about 100 ng/ml of mouse − human chimera monoclonal antibody in 10 ml of the supernatant of their culture medium.

Cell Sorter Analysis of HMH Cells and P3U1 by the Use of Anti − Human IgG

HMH cells and the plastocytoma P3U1 are washed twice in Hank's balanced salt solution (Gibco). The cells ($10^7$) are put in a solution composed of 750 µl of staining buffer solution and 250 µl of rabbit anti −

5

human immunogloblin antibody or 1 μg/ml of normal rabbit IgG. The solution is incubated at room temperatures for one hour. After that, the cells are washed three times in Hank's balanced salt solution. Procedures thereinafter are the same as those written in the avidine – biotine kit, put on the market by Vector Laboratory Co., Ltd.

In brief, the cells are put in 250 μl of a 1/200 human IgG – treated, biotine – conjugated anti – rabbit IgG (1.5 mg/ml) and is incubated at room temperatures for one hour. Subsequently, it is washed three times with Hank's solution, put in 250 μl of a 1/20 avidine FITC (5 mg/ml), and then incubated at room temperatures for 30 minutes.

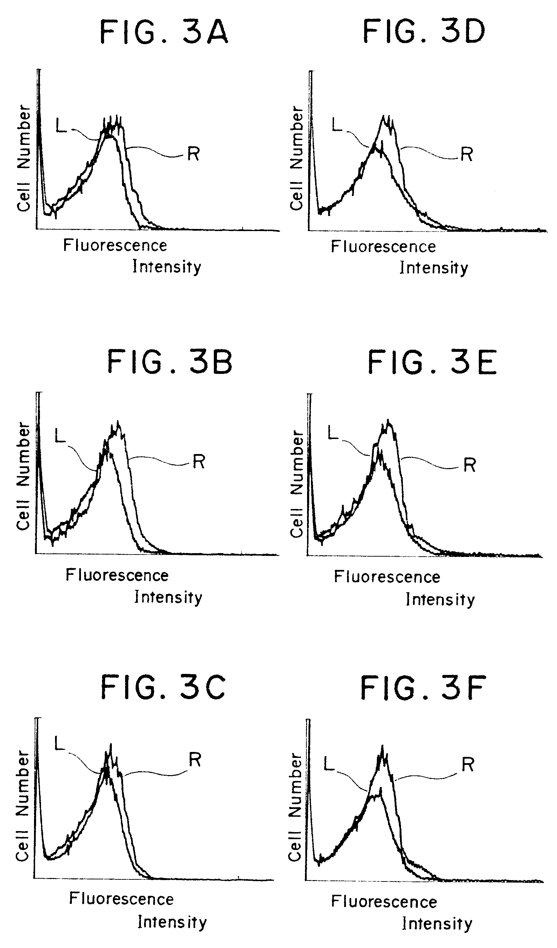

Again, it is washed three times with Hank's solution. Finally, $10^6$ cells are put in 1 ml of a culture medium and subjected to the analysis by the fluorescence – activated cell sorter (FACS IV) equipped with a logarithmic amplifier, manufactured by Beckton – Dickson Co., Ltd. The result is shown in Fig. 3.

In the FACS analysis, HMH cells are used as target cells and rabbit IgG is used as control. That is, in Fig. 3, (a) shows HMH cells reacted with rabbit anti – human Ig antibody, (b) reacted with rabbit anti – human $_x$ antibody, and (c) reacted with rabbit anti – human IgGFc antibody. Likewise, (d) shows the FITC profiles of the composite HMH cells (R) and P3U1 (L) treated with rabbit anti – human Ig antibody, (e) with rabbit anti – human $_x$ chain antibody and (f) with rabbit anti – human IgGFc antibody.

Analysis of DNA Introduced into HMH Cells

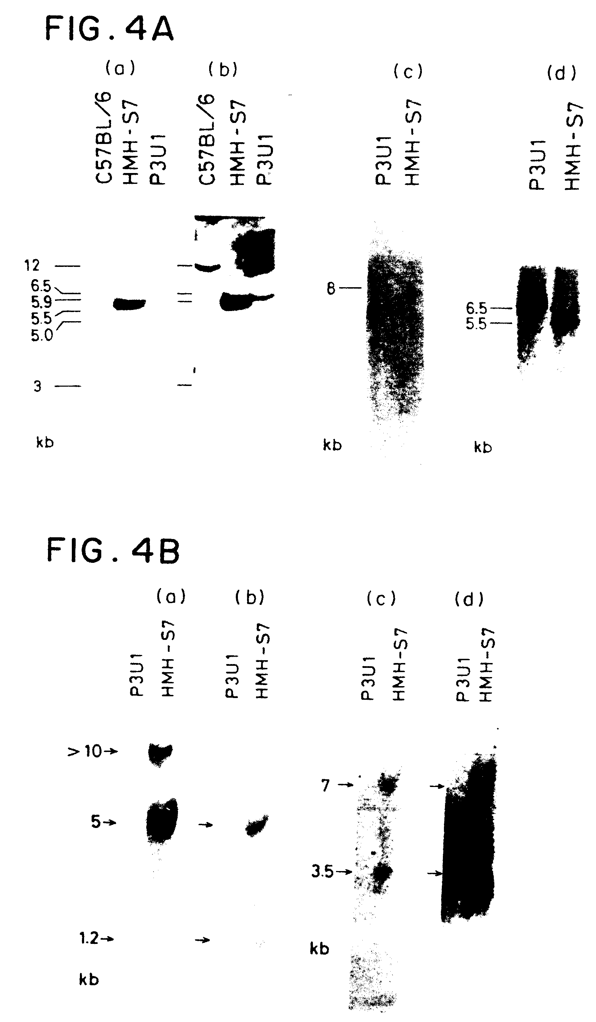

In accordance with the aforementioned method, DNA and Poly A – containing RNA are isolated from HMH cells. Like DNA isolated from HMH cells, DNA isolated from C57BL/6 mouse kidney cells and P3U1 cells is digested with BamHI and subjected to the southern hybridization by means of mouse $J_x$ probe, human $C_x$ probe, mouse $J_H$ probe, and human $C_\gamma$ probe. The result is shown in Figs. 4(a), (b), (c) and (d).

With regard to human $C_x$ and mouse $J_x$ probes, a 5.9 Kb band which corresponds to the size of a BamHI – inserted $pSV2 – HC_kV_{D10}$ is detected in DNA isolated from HMH cells. With regard to mouse $J_H$ probe, a 5.5 Kb band which corresponds to the size of a EcoRI – inserted $pSV2 – HGIV_{D10}$ fragment that contains $V_H$ gene is detected in DNA from HMH cells. With regard to human $C_{\gamma1}$ probe, a EcoRI – BamHI – inserted $pSV2 – HGIV_{D10}$ fragment that contains $C_{\gamma1}$ gene is detected in DNA from HMH cells.

According to these facts, it is obvious that HMH cells possess both heavy and light intact chimena genes in the genome.

Poly A containing m RNAs isolated – from HMH cells and P3U1 are caused to make northern blotting with $VJ_x$ 14 probe, human $C_x$ probe, $VJ_H$ 243 probe and human $C_{\gamma1}$ probe, respectively. With the aid of $VJ_x$ 14 probe and human $C_x$ probe, a 1.2 Kb band which is regarded to have the same size as $x$ chain – producing cells is detected as m RNA of L chain in chimera antibody.

A 5 Kb band is detected as m RNA primary transcript, from which intron in not removed because splicing has not occurred yet. (See Figs. 4B(a) and (b).) Two bands are detected at 3.5 and 7 Kb with the aid of $VJ_H$ 243 and $C_1$ probe as to m RNA of HMH cells. Dimensionally speaking, the former corresponds to m RNA of the membrane – bound type H chain $\gamma$ and the latter corresponds to m RNA from which intron has not been removed yet because it was primary transcript.

As best seen from the above, it becomes evident that in portions of primary transcripts RNA splicing does occur between mouse – derived V – (D) – J exons and human – derived exons in HMH cells.

Although the invention has been described in its preferred from with a certain degree of particularity, it is understood that the present disclosure of the preferred form has been changed in the details of construction and the combination and arrangement of parts may be resorted to without departing from the spirit and the scope of the invention as hereinafter claimed.

Claims

1. A production process of chimera monoclonal antibody consisting of a variable region derived from mouse and a constant region derived from man, which is characterized by comprising (a) inserting into an expression vector active genes: $V_H$ and $V_L$, isolated from hybridomas as antibody – producing cells of mouse and genes: $C_H$ and $C_L$, isolated from human DNA, and then (b) introducing an expression vector from (a) into lymphoma as cultured animal cells.

2. A production process of a chimera monoclonal antibody according to claim 1, wherein the said vector is pSV2 – gpt, pSV2 – neo or SV40.

**Patentansprüche**

1. Verfahren zur Herstellung von chimären monoklonalen Antikörpern, bestehend aus einer von einer Maus abgeleiteten variablen Region und einer vom Menschen abgeleiteten konstanten Region, dadurch **gekennzeichnet,** daß man (a) in einen Expressionsvektor die aktiven Gene $V_H$ und $V_L$, isoliert aus Hybridomen als Antikörper erzeugende Zellen der Maus, und die Gene $C_H$ und $C_L$, isoliert aus menschlicher DNA, insertiert, und dann (b) einen Expressionsvektor aus (a) in ein Lymphom als gezüchtete tierische Zellen einschleust.

2. Verfahren zur Herstellung von chimären monoklonalen Antikörpern nach Anspruch 1, dadurch **gekennzeichnet**, daß der Vektor pSV2−gpt, pSV2−neo oder SV40 ist.

**Revendications**

1. Procédé de production d'un anticorps monoclonal chimérique consistant en une région variable provenant de la souris et une région constante provenant de l'homme, qui est caractérisé en ce qu'il comprend : (a) l'insertion dans un vecteur d'expression des gènes actifs $V_H$ et $V_L$ isolés à partir des hybridomes en tant que cellules de souris produisant des anticorps, et des gènes $C_H$ et $C_L$, isolés à partir d'ADN humain, et ensuite, (b) l'introduction d'un vecteur d'expression provenant de (a) dans un lymphome en tant que cellules animales cultivées.

2. Procédé de production d'un anticorps monoclonal chimérique suivant la revendication 1, dans lequel ce vecteur est pSV2−gpt, pSV2−néo ou SV40.

# FIG. IA

(a)     (b)     (c)     (d)

C57BL/6
D10
P3U1

Cκ probe
VJκI4

C57BL/6
D10
P3U1

Cμ probe
VJH243

kb
6.5→
6.3→
6.1→

kb
1.2→

2.7→

kb
6.5→
5.5→

kb
2.4→

# FIG. IB

(a)  H    B    B    E                    H     VJκI4
                          VκJ2 J3 J4 J5└─┘
                                        Iκb

(b)  E  B      B      B          E        VJH243
                VHD JiJ2J3J4      En

(c)  E      E                EPE  EP      HCκ2
                    Jκ         EnCκ
                              └─┘
                              Iκb

(d)  H              BH                    HGI63
        Cγl

8

# FIG. 2A

# FIG. 2B

## FIG. 3A

Cell Number

L

R

Fluorescence
Intensity

## FIG. 3D

Cell Number

L

R

Fluorescence
Intensity

## FIG. 3B

Cell Number

L

R

Fluorescence
Intensity

## FIG. 3E

Cell Number

L

R

Fluorescence
Intensity

## FIG. 3C

Cell Number

L

R

Fluorescence
Intensity

## FIG. 3F

Cell Number

L

R

Fluorescence
Intensity

# FIG. 4A

# FIG. 4B